# FASCICULE DE BREVET EUROPEEN

(11) **EP 3 471 683 B1**
(45) Date de publication et mention de la délivrance du brevet: **09.06.2021**
(21) Numéro de dépôt: 17735217.6
(22) Date de dépôt: 15.06.2017
(51) Int. Cl.: A61H 33/14

(54) **SYSTÈME APTE À DÉLIVRER UNE PRESSION VARIABLE POUR UNE ENCEINTE DE TRAITEMENT D'UNE PLAIE**
SYSTEM ZUR BEREITSTELLUNG VON VARIABLEM DRUCK FÜR EIN GEHÄUSE ZUR BEHANDLUNG EINER WUNDE
SYSTEM FOR DELIVERING A VARIABLE PRESSURE FOR AN ENCLOSURE FOR TREATMENT OF A WOUND

(30) Priorité: 20.06.2016 FR 1655720
(43) Date de publication de la demande: 24.04.2019
(73) Titulaire: DTAMedical, 39300 Loulle (FR)
(72) Inventeur: DUFAY, François, 39300 Loulle (FR)
(74) Mandataire: Cabinet Beau de Loménie
(86) Numéro de dépôt international: PCT/FR2017/051560
(87) Numéro de publication internationale: WO 2017/220889

(56) Documents cités:
- EP-A1- 2 279 721
- JP-A- 2005 058 745
- US-A1- 2005 191 372
- US-A1- 2012 065 576
- US-A1- 2012 232 467

## Description

La présente invention concerne un système de génération d'un mélange gazeux pour une enceinte de traitement d'une plaie.

Pour un traitement d'une plaie, il est connu d'utiliser une enceinte de traitement soumettant la plaie à un mélange gazeux accélérant la guérison.

Des exemples de générateurs de mélange gazeux sont notamment décrits dans les documents US 2012/065576, US 2012/232467, EP 2 279 721, JP 2005-058745 et US 2005/0191372. Ces générateurs de mélanges gazeux comportent une enceinte de traitement munie d'une entrée et d'une sortie, d'une source de gaz et une unité de contrôle des conditions dans l'enceinte.

Cette disposition donne satisfaction en ce que la plaie est maintenue dans une zone de traitement présentant un environnement contrôlé.

Toutefois, la gestion de la composition du mélange gazeux et son mode de diffusion sur la plaie peuvent s'avérer délicats à mettre en œuvre.

En particulier, même si la seule action des composants du mélange gazeux est bénéfique pour la guérison de la plaie, ces derniers ne permettent pas toujours une stimulation efficace de la circulation sanguine et/ou lymphatique. Des soins complémentaires peuvent s'avérer nécessaires pour la guérison.

La présente invention vise à résoudre tout ou partie des inconvénients mentionnés ci-dessus.

A cet effet, la présente invention concerne un système de génération d'un mélange gazeux pour une enceinte de traitement d'une plaie tel que décrit dans la revendication 1.

Le système de génération de mélange gazeux permet ainsi, grâce à la modulation temporelle de la pression et de la composition du mélange gazeux, de soumettre la plaie à un environnement favorable à sa cicatrisation.

La circulation lymphatique et la circulation sanguine dans les tissus endommagés de la plaie sont ainsi stimulées. La circulation lymphatique permet également d'évacuer les déchets provenant du processus d'inflammation nécessaire à la cicatrisation de la plaie.

Egalement, la variation de la pression permet de stimuler les tissus sans les altérer quelle que soit la surface à traiter : de manière non exhaustive plane pour un ulcère variqueux, en relief pour un pied diabétique.

La gestion de la pression réalisée par le système de génération permet donc de traiter différentes pathologies de plaies aiguës ou chroniques, de manière non exhaustive : plaies du membre supérieur, ulcère variqueux, plaies thoraciques, abdominales, escarres et brûlures nécessitant une pressothérapie.

Selon un aspect de l'invention, le générateur de mélange gazeux comprend un dispositif de recirculation agencé pour recevoir au moins une partie du mélange gazeux ayant traversé l'enceinte de traitement et pour l'incorporer dans le mélange gazeux délivré à l'enceinte de traitement.

Le système de génération est un système semi-ouvert ou fermé permettant d'économiser certaines ressources comme le dioxygène ou la vapeur d'eau.

Selon un aspect de l'invention, le dispositif de recirculation comprend un filtre apte à filtrer l'au moins une partie du mélange gazeux ayant traversé l'enceinte de traitement. Cette disposition permet de limiter la recirculation de déchets ou d'organismes provenant de la plaie.

De préférence, le dispositif de recirculation comprend un déshumidificateur et/ou un humidificateur. Cette disposition permet de faire varier la fraction molaire de vapeur d'eau comprise dans le mélange gazeux délivré.

Selon un aspect de l'invention, le générateur de mélange gazeux est apte à délivrer dans l'enceinte de traitement ledit mélange gazeux à un degré d'hygrométrie déterminé.

Cette disposition permet de réguler le degré d'hygrométrie indépendamment de la pression et de la fraction molaire de dioxygène présent dans le mélange gazeux.

Selon un aspect de l'invention, le générateur de mélange gazeux est configuré pour délivrer dans l'enceinte de traitement ledit mélange gazeux à un degré d'hygrométrie déterminé.

Selon un aspect de l'invention, le processeur est apte à recevoir une information relative à un degré d'hygrométrie interne à l'enceinte de traitement et/ou agencé pour déterminer un degré d'hygrométrie dudit mélange gazeux à délivrer dans l'enceinte de traitement pour atteindre un degré d'hygrométrie interne cible.

Il est donc possible de moduler le degré d'hygrométrie du mélange gazeux délivré et/ou de réguler le degré d'hygrométrie à partir d'une valeur mesurée dans l'enceinte de traitement ou à partir d'une valeur estimée par exemple par calcul ou corrélation avec d'autres valeurs connues ou mesurées.

Selon un aspect de l'invention, la fonction de variation temporelle de la fraction molaire de vapeur d'eau ou du degré d'hygrométrie est une fonction périodique, de préférence ladite fonction périodique est de type sinusoïdal, triangulaire ou présentant des échelons.

Cette disposition permet une variation régulière pour un traitement de la plaie en continu pendant une période longue de plusieurs heures à plusieurs mois.

Selon un aspect de l'invention, le générateur de mélange gazeux est apte à délivrer dans l'enceinte de traitement ledit mélange gazeux à une température déterminée.

Cette disposition permet de s'affranchir des conditions environnant l'enceinte de traitement, notamment la température ambiante. En effet, si l'enceinte se trouve dans une pièce où la température est trop fraiche ou trop élevée, ceci n'a pas d'importance. La plaie est toujours soumise au mélange gazeux à la température optimale pour permettre une guérison rapide de la plaie.

Selon un aspect de l'invention, le générateur de mélange gazeux est configuré pour délivrer dans l'enceinte de traitement ledit mélange gazeux à une température déterminée.

Selon un aspect de l'invention, le processeur est apte à recevoir une information relative à une température interne de l'enceinte de traitement et/ou est agencé pour déterminer une température dudit mélange gazeux à délivrer dans l'enceinte de traitement pour atteindre une température interne cible.

Selon un aspect de l'invention le processeur est agencé pour déterminer une fonction de variation temporelle de la température du mélange gazeux délivré.

Ainsi la température du mélange gazeux à délivrer peut être déterminée et/ou régulée. Cette disposition permet notamment à un utilisateur de se déplacer avec le système de génération et l'enceinte de traitement, par exemple à l'extérieur, sans avoir à réaliser de nouveau réglage.

Egalement, les caractéristiques du matériau constituant l'enceinte ne sont pas limitées à des matériaux thermiquement isolants.

Selon un aspect de l'invention, ladite température interne cible est entre 28°C et 43°C, de préférence entre 28 et 40°C. Ainsi le système de génération permet de soumettre la plaie à différentes température selon les pathologies soignées.

Selon un autre aspect de l'invention, ladite température interne cible est inférieure à 28°C.

Selon un aspect de l'invention, le processeur est agencé pour déterminer un programme de traitement comprenant au moins une phase de fonctionnement selon un mode de fonctionnement tel que le traitement de la phase inflammatoire, traitement de la phase de bourgeonnement, traitement de la phase d'épithélialisation.

Le programme de traitement est donc défini selon l'état de la plaie. Même si trois modes de fonctionnement existent, un nombre plus élevé de programmes de traitement peut être mis au point car chaque programme comprend l'application successive de plusieurs modes de fonctionnement, chacun ayant une durée spécifique.

Selon un aspect de l'invention, la fonction de variation temporelle de la pression est une fonction périodique.

Cette disposition permet une alternance de la pression soumise à la peau : cette alternance a des propriétés thérapeutiques.

Selon un aspect de l'invention, ladite fonction périodique est de type sinusoïdal, triangulaire ou présentant des échelons.

Selon un aspect de l'invention, le générateur de mélange gazeux est agencé pour maintenir la pression interne à l'enceinte de traitement constante pendant une durée déterminée.

Ainsi la plaie peut être maintenue en pression pendant une durée déterminée. Cette disposition est utile par exemple pour tenter de limiter certaines complications de la cicatrisation.

Selon un aspect de l'invention, la fonction de variation temporelle de la pression comprend une phase de montée en pression et une phase de descente de pression, la durée de chaque phase sera fonction de l'effet thérapeutique recherché..

Selon un aspect de l'invention, une phase de maintien en pression succède à la phase de montée en pression et précède la phase de descente.

Selon un aspect de l'invention, la pression cible est comprise entre 0 et 600 mmHg au dessus de la pression ambiante externe à l'enceinte de traitement.

Selon un aspect de l'invention, la pression cible est comprise entre 0 et 300 mmHg au dessus de la pression ambiante externe à l'enceinte de traitement.

Selon un aspect de l'invention, la pression cible est comprise entre 30 et 40 mmHg au dessus de la pression ambiante externe à l'enceinte de traitement aura un effet thérapeutique adapté à certaines situations de plaies.

Selon un aspect de l'invention, la pression cible est comprise entre 45 et 60 mmHg au dessus de la pression ambiante externe à l'enceinte de traitement. Cette disposition permet en particulier d'accompagner le traitement des brûlures et pourra être intéressante thérapeutiquement dans certaines zones anatomiques.

Selon un aspect de l'invention, la pression cible est comprise entre 200 et 300 mmHg au dessus de la pression ambiante externe à l'enceinte de traitement. Ces pressions peuvent être utiles pour traiter des affections sévères.

Selon un aspect de l'invention, la fraction molaire de dioxygène est comprise entre 0 et 1.

Selon un aspect de l'invention, le processeur est apte à recevoir une information relative à une fraction molaire de dioxygène interne à l'enceinte de traitement et/ou agencé pour déterminer une fraction molaire de dioxygène dudit mélange gazeux à délivrer dans l'enceinte de traitement pour atteindre une fraction molaire de dioxygène interne cible.

Il est donc possible de moduler la fraction molaire de dioxygène du mélange gazeux délivré et/ou de réguler la fraction molaire de dioxygène à partir d'une valeur mesurée dans l'enceinte de traitement ou estimée.

Cette disposition permet de faire varier la fraction molaire de dioxygène de manière significative pour stimuler la plaie.

Selon un aspect de l'invention, la fonction de variation temporelle de dioxygène comprend une phase ascendante de la fraction molaire de dioxygène et une phase descendante de la fraction molaire de dioxygène.

Cette variation de la fraction molaire de dioxygène favorisera le processus de cicatrisation
Selon un aspect de l'invention, la fonction de variation temporelle de la fraction molaire de dioxygène est une fonction périodique, en particulier ladite fonction périodique est de type sinusoïdal, triangulaire ou présentant des échelons.

Selon un aspect de l'invention, le mélange gazeux comprend en outre par exemple une fraction molaire de diazote et/ou une fraction molaire de dioxyde de carbone et/ou un autre gaz adapté comme le protoxyde d'azote ou autres gaz rares.

Cette disposition permet au générateur de mélange gazeux de réguler aisément la fraction molaire de dioxygène et la fraction molaire de vapeur d'eau. Selon un aspect de l'invention, le mélange gazeux comprend de l'air.

Selon un aspect de l'invention, le processeur agencé pour déterminer une fonction de variation temporelle de la fraction molaire de diazote et/ou une fonction de variation temporelle de la fraction molaire de dioxyde de carbone et/ou de l'autre gaz adapté dudit mélange gazeux à délivrer dans l'enceinte de traitement.

Cette disposition permet de faire diminuer la fraction molaire de dioxygène et/ou la fraction molaire de vapeur d'eau dans le mélange gazeux..

La fraction molaire de dioxyde de carbone permet également de réguler le pH du mélange gazeux et ainsi obtenir un pH adapté au stade de la cicatrisation.

Selon un aspect de l'invention, le système de génération comprend un dispositif d'adjonction d'un gaz additionnel et/ou d'un aérosol dans le mélange gazeux délivré par le générateur de mélange gazeux, le dispositif d'adjonction est agencé pour nébuliser ledit aérosol et/ou vaporiser et/ou sublimer ledit gaz additionnel dans le mélange gazeux délivré par le générateur de mélange gazeux.

La simultanéité d'une part de la variation des fractions molaires et d'autre part de l'adjonction d'un composant additionnel a un effet bénéfique sur la plaie. Le composant additionnel est diffusé sous forme d'un gaz additionnel et/ou d'un aérosol.

Par aérosol il est entendu ensemble de particules fines liquides et/ou solides.

Selon un aspect de l'invention, le gaz additionnel et/ou l'aérosol comprend un principe actif destiné à soigner la plaie.

Ce principe de diffusion de principe actif de manière simultanée avec la variation de la composition du mélange gazeux est apte à accompagner au mieux le processus de cicatrisation.

Cette disposition permet d'obtenir un mélange gazeux uniforme enrichi en gaz additionnel et/ou en aérosol. De préférence, le gaz additionnel et/ou l'aérosol comprennent un principe actif apte à avoir un effet sur la plaie.

Au sein de l'enceinte de traitement, la plaie est donc baignée dans un mélange gazeux comprenant un principe actif. Puisque l'exposition à ce principe actif est réalisée en continu selon une période assez longue allant de quelques heures à quelques mois, la plaie est dans de bonnes conditions pour cicatriser.

Selon un aspect de l'invention, l'unité de contrôle comprend une interface utilisateur pourvue d'une commande utilisateur et/ou d'un organe de retour comprenant un écran et/ou un voyant.

L'utilisateur est donc apte à influer sur le réglage de l'unité de contrôle. En particulier l'utilisateur peut créer, modifier et/ou sélectionner une fonction de variation temporelle de la pression, de la température ou de la fraction molaire des constituants du mélange gazeux.

Selon un aspect de l'invention, Le processeur comprend en mémoire une pluralité de fonctions de variation temporelle de la pression, de la température et de la fraction molaire des constituants du mélange gazeux, la commande utilisateur étant agencée pour qu'un utilisateur sélectionne au moins une fonction de variation temporelle à appliquer par le système de génération.

L'utilisation du système de génération est donc aisée : l'utilisateur doit sélectionner la ou les fonctions de variation temporelles correspondant à l'état et au type de la plaie à traiter.

Selon un aspect de l'invention, l'interface utilisateur comprend un verrouillage agencé pour limiter la sélection et/ou la création et/ou la modification d'au moins une fonction de variation temporelle par une clé. De préférence ladite clé est un code d'accès.

Cette disposition permet de définir différents niveaux de gestion du système de génération : par exemple un médecin peut avoir le code d'accès pour sélectionner n'importe quelle fonction de variation temporelle, tandis que le nombre de fonction accessible est limitée pour une infirmière et encore plus limité pour le patient.

Ainsi il est possible de s'assurer que le traitement sera correctement suivi tout en laissant une marge d'adaptabilité du dispositif en fonction des circonstances.

L'organe de retour quant à lui permet de connaitre des données de retour en provenance de l'enceinte, par exemple des valeurs mesurées ou estimées. Les valeurs estimées peuvent provenir de calculs ou de corrélations réalisées à partir d'autres valeurs connues ou mesurées dans l'enceinte ou dans l'environnement de l'enceinte.

Il est donc possible au médecin de contrôler si un traitement a bien été appliqué lorsqu'il consulte l'historique des données de retour en provenance de l'enceinte ou de l'environnement de l'enceinte.

Selon un aspect de l'invention, l'interface utilisateur comprend une prise de transfert d'information par exemple une prise USB. Cette disposition permet à l'utilisateur de transférer l'ensemble de l'historique des traitements réalisés, par exemple sous forme de tableau de valeurs. Il est alors possible d'analyser l'évolution de la plaie à partir de ces données.

De manière alternative la prise peut être remplacée par un émetteur apte à transmettre des informations selon un protocole connu, par exemple filaire ou par ondes radio.

Selon un aspect de l'invention, le système de génération d'un mélange gazeux comprend un système de mesure agencé pour mesurer une oxymétrie transcutanée de la plaie et agencé pour transmettre des valeurs de mesures au processeur.

Cette disposition permet de renseigner davantage le processeur sur l'état de la plaie en déterminant un état réel de l'état d'oxygénation des tissus de la plaie.

De préférence, le système de mesure est agencé pour mesurer une pression transcutanée pour le diazote et/ou le dioxyde de carbone au niveau de la plaie. L'oxymétrie transcutanée ainsi que la pression transcutanée sont également connues sous les dénominations TCPO2, TCPN2, TCPCO2.

Selon un aspect de l'invention, le système de mesure comprend en outre un dispositif de capillaroscopie et/ou un capteur de température de la plaie. Cette disposition permet de renseigner davantage le processeur sur l'état de la plaie.

Plus une plaie est perfusée par du sang artériel oxygéné, plus la plaie sera chaude : l'inflammation signifie que le sang artériel amène tous les éléments nécessaires à la réparation des tissus d'où l'augmentation de la température.

Selon un aspect de l'invention, le système de mesure est en outre agencé pour mesurer une fluorescence par angiographie, par prise de tension au niveau d'artérioles de la plaie, et/ou comprend des dispositifs de mesure par laser doppler et/ou iontophorèse.

Ainsi divers capteurs peuvent être adaptés sur l'appareil de traitement pour identifier au mieux l'état de la plaie.

Selon un aspect de l'invention, le processeur est agencé pour recevoir une information relative à une dimension et/ou à une phase d'évolution d'au moins une partie d'une plaie.

Cette disposition permet au processeur d'adapter les paramètres du mélange pour que le traitement de la plaie soit optimal et adaptatif. Puisque cet ajustement est réalisé automatiquement par le processeur, il n'y a pas de risque de mauvais réglages de la part de l'utilisateur.

Selon un aspect de l'invention, ladite information relative à la dimension et/ou à la phase d'évolution, pour l'au moins une partie de la plaie, comprend une aire et/ou une phase d'évolution parmi lesquelles une phase inflammatoire, une phase de bourgeonnement et une phase d'épithélialisation.

Ainsi la taille de la plaie observée peut être constante et celle-ci peut comprendre plusieurs parties dont l'aire et/ou la phase d'évolution changent.

Cette disposition permet donc au processeur d'adapter les fonctions de variation temporelle à la taille de la plaie et/ou de ses différentes parties constituantes. Non seulement le traitement proposé par le système de génération permet de cibler un type de plaie déterminé, mais également de s'adapter à la taille de la plaie et/ou de ses différentes parties constituantes.

Selon un aspect de l'invention, l'unité de contrôle comprend un capteur apte à déterminer ladite information relative à la dimension et/ou à la phase d'évolution d'au moins une partie de la plaie. Ledit capteur comprend une caméra.

Cette disposition permet une adaptation constante du traitement proposé à la plaie.

Selon un aspect de l'invention, la caméra est disposée sur un bras du système de génération, la caméra étant apte à être déplacée pour réaliser une prise de vue d'au moins une partie de la plaie.

Selon un aspect de l'invention, la caméra est ménagée dans l'enceinte de traitement. Cette disposition permet d'automatiser la prise d'image car la caméra est fixe par rapport à l'enceinte de traitement.

Selon un aspect de l'invention, le capteur apte à déterminer ladite information relative à la dimension et/ou à la phase d'évolution d'au moins une partie de la plaie comprend au moins deux caméras.

De préférence, les au moins deux caméras sont ménagées dans l'enceinte de traitement. Cette disposition permet de réaliser une reconstruction tridimensionnelle de la plaie dans le but d'obtenir des informations plus détaillées concernant l'état de la plaie.

Selon un aspect de l'invention, la commande utilisateur est agencée pour qu'un utilisateur saisisse, sélectionne et/ou modifie ladite information relative à la dimension et/ou à la phase d'évolution.

Cette disposition permet une définition manuelle de la taille et de l'évolution de la plaie. Par exemple un médecin peut la mettre à jour lors de sa consultation.

Selon un aspect de l'invention, le processeur est agencé pour conserver en mémoire au moins une information relative à la dimension et/ou à la phase d'évolution de la plaie. De préférence, le processeur est agencé pour conserver en mémoire un historique des modifications de ladite au moins une information.

Selon un aspect de l'invention, le processeur comprend un module de traitement agencé pour déterminer une phase d'évolution de tout ou partie de la plaie, la détermination étant réalisée à partir de l'information relative à une dimension et/ou à une phase d'évolution d'au moins une partie d'une plaie.

Cette disposition permet de compléter l'analyse visuelle de la plaie réalisée par un médecin : non seulement l'unité de commande permet de faire remonter des informations relatives à une dimension et/ou à une phase d'évolution de la plaie, mais encore de traiter ces informations dans le but de déterminer la phase d'évolution de tout ou partie de la plaie.

Selon un aspect de l'invention, le module de traitement est agencé pour déterminer un contour de tout ou partie de la plaie et pour affecter à une zone délimitée par ledit contour une phase d'évolution, l'affectation étant réalisée à partir de l'information relative à une dimension et/ou à une phase d'évolution d'au moins une partie d'une plaie.

Cette disposition permet de déterminer les contours de différentes parties de la plaie selon leurs phases d'évolution.

Ainsi, cette disposition permet non seulement d'utiliser des informations mesurées mais également d'en déduire des caractéristiques de la plaie en croisant ces informations.

Selon un aspect de l'invention, le contour est déterminé par une méthode de segmentation type de ligne de partage des eaux ou de croissance de régions, ladite méthode étant mise en œuvre par le module de traitement.

Les méthodes ou algorithmes appliqués par le module traitement sont fiables et limitent les besoins en ressources de calculs de la part du processeur par rapport à la précision obtenue. Ceci permet une bonne identification de l'état de la plaie.

Selon un autre aspect de l'invention, le module de traitement est agencé pour comparer l'information relative à une dimension et/ou à une phase d'évolution d'au moins une partie d'une plaie à une pluralité d'informations de définitions de phases d'évolution pour déterminer la phase d'évolution de tout ou partie de la plaie.

Cette disposition permet de ne pas avoir à déterminer de contour. En effet la comparaison aux informations de définition permet de comparer la plaie dans son ensemble à une définition d'un stade connu.

Selon un autre aspect de l'invention, la comparaison est réalisée selon une méthode de motifs binaires locaux ou local binary patterns en anglais. L'utilisation de cette méthode permet de s'affranchir de l'étape de segmentation et ainsi d'améliorer la robustesse de la méthode.

Selon un aspect de l'invention, une phase d'évolution est définie par une pluralité d'éléments descriptifs, notamment la couleur et la texture de la zone délimitée par le contour.

Cette disposition permet de définir avec précision la phase d'évolution en prenant en compte toutes les étapes successives de changement d'état de la plaie.

Selon un aspect de l'invention, la comparaison est réalisée selon une méthode de régression des moindres carrés partiels de Kernel ou KPLS, ladite méthode étant mise en œuvre par le module de traitement et le résultat étant une probabilité d'appartenance à une phase d'évolution.

Cette disposition permet de déterminer à partir d'informations mesurées l'état le plus probable de la plaie. Ainsi la détermination est rapide et fiable.

Selon un autre aspect de l'invention, la phase d'évolution est déterminée par une méthode de classification, notamment Software Vector Machine ou Deep Learning.

Cette disposition permet de prévoir des alternatives possibles d'identification de la phase d'évolution.

Selon un aspect de l'invention, le module de traitement est agencé pour mettre en œuvre une phase initiale d'apprentissage pendant laquelle une pluralité d'informations de définition sont transmises au module de détermination avec pour chaque information une correspondance avec une phase d'évolution donnée.

Cette disposition permet une calibration du module de traitement, la calibration pouvant ainsi être adaptée selon le type de matériel utilisé pour les mesures et/ou selon le type de plaie soignée.

Selon un aspect de l'invention, le module de traitement est agencé pour déterminer une consigne de commande définissant la fonction de variation temporelle de la pression et/ou la fonction de variation temporelle de la fraction molaire de dioxygène.

Le module de traitement permet ainsi suite à l'analyse des valeurs mesurées de déterminer par calcul la consigne de commande la plus adaptée à l'état de la plaie.

Selon un aspect de l'invention, la consigne de commande définit pour le mélange gazeux un degré d'hygrométrie, une température, une fraction molaire de vapeur d'eau, une fraction molaire de diazote, une fraction molaire de dioxyde de carbone, et/ou une fraction de gaz additionnel ou aérosol.

De préférence, l'interface utilisateur est configurée pour soumettre à une validation de l'utilisateur la consigne de commande déterminée par le module de traitement.

Ainsi la consigne de commande est à considérer comme une proposition faite au médecin responsable du traitement qui peut ensuite éventuellement modifier la consigne de commande si son analyse de l'état de la plaie est différente.

Selon un autre aspect de l'invention, l'unité de contrôle est agencée pour appliquer automatiquement la consigne de commande après sa détermination. Cette disposition permet d'automatiser le traitement de la plaie en adaptant le traitement à la réaction de la plaie.

Selon un aspect de l'invention, le module de traitement est agencé pour déterminer au moins deux consignes de commande, de préférence un niveau de pertinence étant affecté à chaque consigne de commande.

Cette disposition permet au médecin de choisir le traitement qui lui semble le meilleur pour la plaie.

Selon un aspect de l'invention, le système de génération est agencé pour être accroché à une potence de perfusion et/ou à un bandeau de lit.

Cette disposition permet de déplacer facilement le système de génération. Ainsi l'utilisateur qui traite sa plaie peut se déplacer avec le système de génération.

La fonction ambulatoire est particulièrement intéressante pour les plaies chroniques de type ulcères variqueux, pied diabétique ou artérite. Le traitement n'empêche pas l'utilisateur de se déplacer.

La présente invention concerne également un appareil de traitement comprenant un système de génération tel que décrit ci-avant et une enceinte de traitement agencée pour être disposée sur et/ou autour d'une partie d'un corps d'un utilisateur de manière à engendrer une zone de traitement.

Selon un aspect de l'invention, l'enceinte de traitement comprend une entrée pour le mélange gazeux délivré par le système de génération dans la zone de traitement.

Selon un aspect de l'invention, l'enceinte de traitement est agencée pour entourer une partie du corps par exemple un membre supérieur ou un pied de l'utilisateur et comprend une semelle pour la marche de l'utilisateur. Cette disposition est intéressante pour le pied diabétique : l'utilisateur devient autonome.

Selon un aspect de l'invention, l'enceinte de traitement comprend une surface de contact apte à coopérer avec la peau d'un utilisateur, la surface de contact étant apte à isoler fluidiquement la zone de l'extérieur.

Selon un aspect de l'invention, la surface de contact est apte à adhérer à la peau de l'utilisateur, de préférence l'enceinte de traitement comprend un produit permettant un maintien isolant la plaie de l'environnement extérieur disposé sur ladite surface de contact.

Cette disposition permet d'isoler fluidiquement l'enceinte de traitement en faisant adhérer la surface de contact à la peau grâce au produit collant.

Selon un aspect de l'invention, l'enceinte de traitement comprend un joint sur lequel est ménagée la surface de contact. De préférence, le joint comprend du silicone.

Selon un aspect de l'invention, le joint comprend une chambre interne présentant des ouvertures communiquant avec la zone de traitement, la sortie et/ou l'entrée de la zone de traitement. Ainsi le joint est maintenu en position par le mélange gazeux pénétrant dans la chambre interne.

Cette disposition permet d'améliorer la tenue de l'enceinte de traitement autour d'un membre de l'utilisateur.

Selon un aspect de l'invention, l'enceinte de traitement comprend une enveloppe externe apte à présenter une forme déterminée lorsque le système de génération délivre le mélange gazeux, l'enveloppe étant apte à se déformer en réponse à une contrainte imposée sur sa surface et reprendre la forme déterminée en l'absence de ladite contrainte.

Cette disposition permet un massage de la plaie puisque la contrainte permet de modifier localement la pression et ainsi déformer localement la peau. Médicalement, le massage de la plaie augmente la possibilité d'échanges entre le mélange gazeux et les tissus de la plaie et améliore la circulation sanguine et la circulation lymphatique, utile pour certains types de plaies.

Cette disposition permet d'augmenter la physiologie de la cicatrisation.

Selon un aspect de l'invention, la fonction de variation temporelle de la fraction molaire de dioxygène et/ou de vapeur d'eau et/ou de dioxyde de carbone et/ou de diazote et/ou de tout autre composant de l'air comprend une phase de montée de ladite fraction molaire et une phase de descente de ladite fraction molaire.

Selon un aspect de l'invention, une phase de maintien de la fraction molaire succède à la phase de montée et précède la phase de descente.

Dans le présent texte, le mélange gazeux délivré peut également être défini par une pression de délivrance en lieu et place du débit délivré. Dans ce cas, une pression partielle de la pression de délivrance peut être définie pour chaque composant. Cette pression partielle d'un composant correspond à la fraction molaire du composant multipliée par la pression de délivrance.

De toute façon l'invention sera bien comprise à l'aide de la description qui suit en référence aux dessins schématiques annexés représentant, à titre d'exemple non limitatif, une forme d'exécution de ce système de génération d'un mélange gazeux.
Figure 1 est une vue en perspective d'un appareil de traitement d'une plaie et d'un utilisateur.
Figures 2 à 8 est une vue en perspective d'une enceinte de traitement de l'appareil de traitement.
Figure 9 est un schéma représentant des phases d'évolution de la plaie.
Figure 10 est une représentation graphique temporelle des phases d'évolution de la plaie.

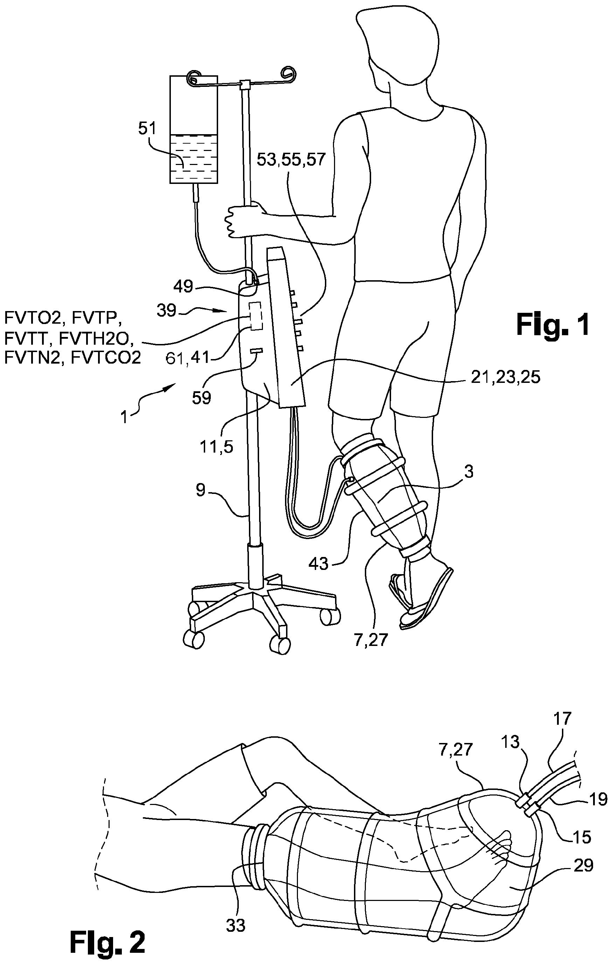

Comme illustré à la figure 1, un appareil de traitement 1 d'une plaie 3 comprend un système de génération 5 d'un mélange gazeux et une enceinte de traitement 7.

Le système de génération 5 est agencé pour être accroché à une potence 9 de perfusion. En outre le système de génération 5 est agencé pour être accroché à un bandeau de lit. L'appareil de traitement 1 peut donc être mobile ou statique selon les contraintes d'utilisation.

Le système de génération 5 comprend un générateur de mélange gazeux 11 apte à délivrer le mélange gazeux dans l'enceinte de traitement 7 à un débit déterminé.

Pour ce faire et comme illustré aux figures 2 à 8, l'enceinte de traitement 7 est pourvue d'une entrée 13 pour le mélange gazeux en provenance du générateur de mélange gazeux 11.

L'enceinte de traitement 7 comprend également une sortie 15. Le mélange gazeux délivré circule donc à l'intérieur de l'enceinte de traitement 7.

Le système de génération 5 comprend une conduite d'entrée 17 reliant le générateur de mélange gazeux 11 à l'entrée 13 de l'enceinte de traitement 7 et une conduite de retour 19 vers un dispositif de recirculation 21 du système de génération 5.

Le dispositif de recirculation 21 est agencé pour recevoir au moins une partie du mélange gazeux ayant traversé l'enceinte de traitement 7 et pour l'incorporer dans le mélange gazeux délivré.

Ainsi il est possible de ne pas faire recirculer le mélange gazeux, par exemple lorsqu'il est nécessaire de changer la composition du mélange gazeux ou de faire recirculer une partie ou totalité du mélange gazeux pour des motifs d'économie de composants du mélange gazeux.

Le dispositif de recirculation 21 comprend en outre un filtre 23 apte à retirer du mélange gazeux des déchets provenant de la plaie 3 et un humidificateur 25 pour adapter un degré d'hygrométrie interne de l'enceinte de traitement.

L'enceinte de traitement 7 comprend une enveloppe externe 27 apte à présenter une forme déterminée lorsque le système de génération 5 délivre le mélange gazeux.

La forme déterminée consiste en un déploiement de l'enveloppe externe 27 puisque une pression P interne à l'enceinte de traitement 7 est supérieure à une pression ambiante externe à l'enceinte de traitement 7 en conditions d'utilisation.

Ainsi dans le présent texte, lorsqu'il est fait référence à une pression P interne de l'enceinte de traitement 7 en conditions de fonctionnement, cette pression P est exprimée comme une pression relative par rapport à la pression ambiante externe.

L'enveloppe externe 27 est également apte à se déformer en réponse à une contrainte imposée sur sa surface et reprendre la forme déterminée en l'absence de ladite contrainte.

Cette disposition permet de réaliser indirectement un massage de la plaie 3 en appuyant sur l'enveloppe externe. En effet, un tel appui modifie localement la pression P interne à l'enceinte de traitement 7 sans qu'il soit nécessaire de toucher la plaie 3 ou la peau autour de la plaie 3.

L'enceinte de traitement 7 est agencée pour être disposée sur et/ou autour d'une partie d'un corps d'un utilisateur de manière à engendrer une zone de traitement 29 dans laquelle circule le mélange gazeux.

L'enceinte de traitement 7 comprend une surface de contact 31 apte à coopérer avec la peau de l'utilisateur, la surface de contact 31 étant apte à isoler fluidiquement la zone de traitement 29 de l'enceinte de traitement 7 de l'extérieur.

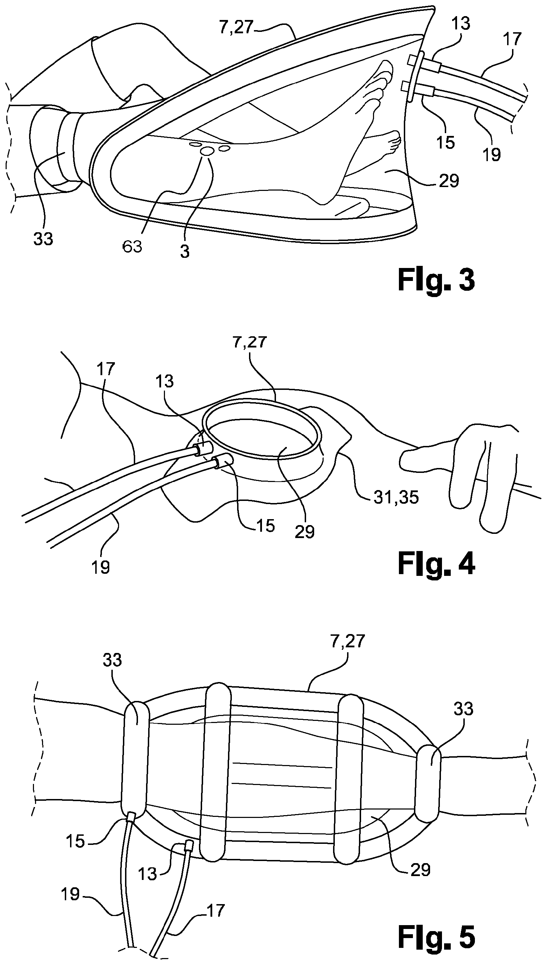

La surface de contact 31 peut être ménagée dans un joint en silicone 33 de l'enceinte de traitement comme présenté aux figures 2, 3, 5, 6 et 8. L'enceinte de traitement 7 comprend également, soit à la place soit en plus du joint en silicone 33, un produit collant 35 disposé sur ladite surface de contact 31. Par exemple ceci est illustré aux figures 4, 6 et 7.

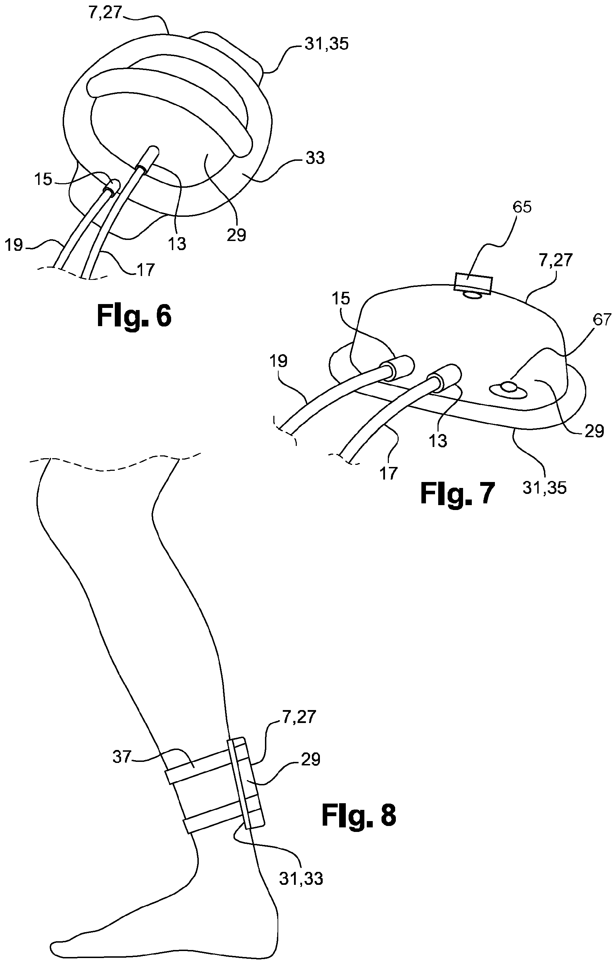

De manière optionnelle, le joint en silicone 31 peut comprendre une chambre interne présentant des ouvertures communiquant avec la zone de traitement et la sortie de la zone de traitement comme illustré aux figures 5 et 6.

Ainsi le fait que la pression P interne soit supérieure à la pression ambiante n'implique pas de fuite de mélange gazeux vers l'extérieur.

A la figure 8, le système de génération 5 comprend en outre des lanières 37 pour maintenir en position l'enceinte de traitement 7.

Le système de génération 5 comprend une unité de contrôle 39 pourvue d'un processeur 41 apte à contrôler le générateur de mélange gazeux 11 et définir un débit, une température et une composition du mélange gazeux à délivrer.

En outre le générateur de mélange gazeux 11 comprend un réservoir de chaque composant du mélange gazeux ou est agencé pour être raccordé à une ou plusieurs bouteilles externes.

Le mélange gazeux comprend une fraction molaire de dioxygène O2 et une fraction molaire de vapeur d'eau H2O. Le mélange gazeux peut également comprendre une fraction molaire de diazote N2, une fraction molaire de dioxyde de carbone CO2 et/ou une fraction molaire de composants de l'air autres que ceux nommés ci-avant.

Le processeur est agencé pour déterminer une fonction de variation temporelle de la fraction molaire de diazote FVTN2 et/ou une fonction de variation temporelle de la de dioxyde de carbone FVTCO2 dudit mélange gazeux à délivrer dans l'enceinte de traitement.

L'unité de contrôle 39 peut comprendre des capteurs 43 pour déterminer une pression P interne, un degré d'hygrométrie DHH2O interne ou une température T interne à l'enceinte de traitement 7.

Ces capteurs 43 permettent d'envoyer respectivement au processeur 41 selon un protocole de communication filaire ou par ondes radio, une information relative à la pression P interne instantanée, au degré d'hygrométrie DHH2O instantané et à la température interne instantanée.

Un des capteurs 43 est une caméra 65 apte à transmettre une information relative à une dimension a1, a2, a3 et à une phase d'évolution a, b, c de la plaie d'au moins une partie de la plaie.

Par dimension a1, a2, a3 il est entendu une aire et par phase d'évolution a, b, c une des trois phases suivantes : inflammatoire a, de bourgeonnement b ou d'épithélialisation c.

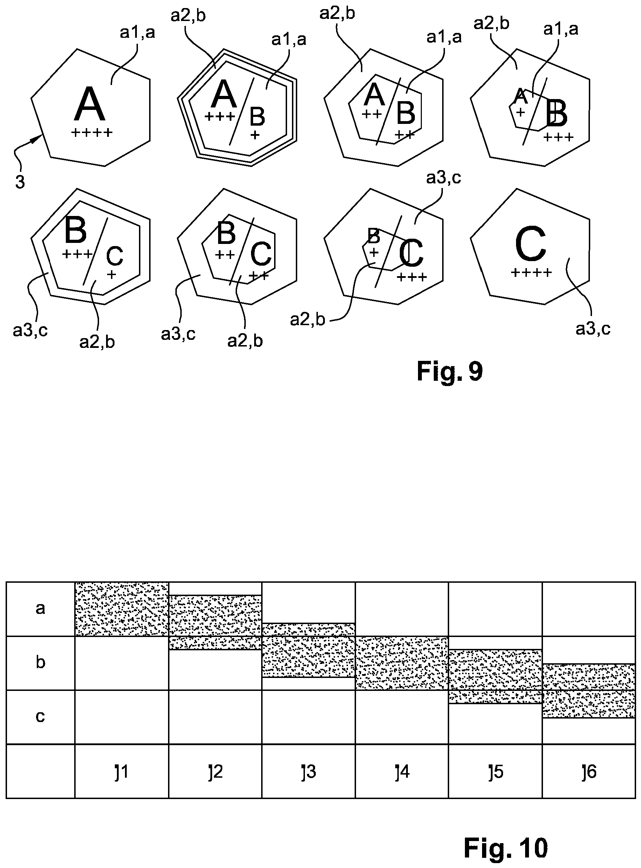

Il est également entendu qu'une plaie peut être définie par une information relative à plusieurs parties d'une plaie, chacune des parties étant définie par une dimension a1, a2, a3 et une phase d'évolution a, b, c. La plaie est en quelque sorte modélisée en plusieurs parties chacune définie par une aire ayant une phase d'évolution propre comme illustré à la figure 9.

Toutes ces informations sont des données d'entrée pour le processeur 41 qui peut définir la composition, le débit et la température du mélange gazeux à délivrer pour qu'un programme de traitement soit appliqué à la plaie 3.

Le processeur 41 est agencé pour définir une température T cible, un degré d'hygrométrie DHH2O cible et une pression P cible à l'intérieur de l'enceinte de traitement 7 et à modifier en conséquence la composition du mélange gazeux délivré dans l'enceinte, sa température et son débit.

Ces valeurs cibles peuvent être constantes mais peuvent également varier en fonction du temps, par exemple sous forme de fonctions périodiques de type sinusoïdal, triangulaire ou présentant des échelons.

Ainsi le processeur 41 est agencé pour définir une fonction de variation temporelle de la pression interne à l'enceinte FVTP, la pression P étant toujours supérieure à la pression ambiante externe à l'enceinte de traitement.

La fonction de variation de la pression FVTP permet d'améliorer la circulation sanguine et dépend du type de plaie 3.

Le processeur 41 est également agencé pour définir une fonction de variation temporelle de la fraction molaire de dioxygène FVTO2 du mélange gazeux délivré, une fonction de variation temporelle de la fraction molaire de vapeur d'eau FVTH2O du mélange gazeux délivré et une fonction de variation temporelle de la température FVTT interne à l'enceinte de traitement 7. A la figure 10 est représentée l'évolution d'une plaie selon les phases a, b, c définies à la figure 9 sur six jours j1 à j6. Les lettres A, B et C sont des modes de fonctionnement appliqués successivement à la plaie selon les phases a, b, c.

Lorsque la plaie comprend plusieurs parties à des phases différentes, par exemple a et b, un programme de traitement comprend une première partie selon un mode puis une deuxième partie selon un autre mode, par exemple A et B. La durée de chaque partie est également modulable.

Le système de génération 5 comprend en outre un dispositif d'adjonction 49 dans le mélange d'un gaz additionnel et/ou un aérosol 51 sous forme nébulisée, vaporisée ou sublimé.

Le gaz additionnel et/ou l'aérosol 51 peut comprendre une substance active pour le traitement de la plaie 3 ou tout type de produit ayant un effet positif sur la guérison de la plaie 3. Par exemple, il peut s'agir d'une solution aqueuse comprenant des principes actifs pour le traitement de la plaie.

Le processeur 41 est également agencé pour faire varier la quantité de gaz additionnel et/ou d'aérosol 51 dans le mélange gazeux.

Le système de génération 5 comprend également une interface utilisateur 53 pourvue d'une commande utilisateur 55 agencée pour permettre à l'utilisateur soit de choisir un programme de traitement soit de modifier manuellement une fonction de variation temporelle FVT.

Chaque modification est rendue possible par la saisie d'une clé de type code d'accès pour passer outre un verrouillage par défaut de la commande utilisateur 53. Selon la clé utilisée, l'utilisateur a accès à plus ou moins de choix.

Ainsi une clé peut être définie pour un médecin, une autre pour une infirmière avec moins de réglages possibles et une autre pour un utilisateur impliquant un accès limité aux réglages.

L'interface utilisateur 51 peut également être utilisée pour définir l'information relative à la dimension a1, a2, a3 et à la phase d'évolution a, b, c de parties de la plaie 3, par exemple lorsque le système de génération ne comprend pas de caméra 65.

L'interface utilisateur 53 comprend également un organe de retour 57 pourvu d'un écran et/ou d'un voyant pour permettre à l'utilisateur de consulter les réglages des fonctions de variations temporelles FVT de pression et les relevés des informations en provenance des capteurs 43.

L'interface utilisateur 53 comprend en outre une prise de transfert 59 d'informations par exemple une prise USB pour transmettre des informations concernant le réglage ou un historique de mesures des capteurs 43 et des informations concernant les programmes de traitement appliqués précédemment à l'utilisateur. Les informations transférées peuvent se présenter sous forme de tableaux de valeurs.

De manière alternative la prise peut être remplacée par un émetteur apte à transmettre des informations selon un protocole connu, par exemple filaire ou par ondes radio.

L'appareil de traitement 1 décrit a l'avantage d'être transportable : l'utilisateur peut donc se déplacer avec : il peut être utilisé sur de très longues périodes comme plusieurs jours ou semaines, sans perturber la vie de l'utilisateur.

Cet appareil de traitement 1 permet également d'adapter en permanence le traitement désiré au type de plaie et à son évolution.

Comme illustré aux figures 1 et 3, le processeur 41 comprend un module de traitement 61 agencé pour déterminer au moins un contour 63 de tout ou partie de la plaie 3.

Ainsi le module de traitement 61 est agencé pour exploiter l'image fournie par la caméra 65 comme illustré à la figure 7, pour déterminer un ou plusieurs contours 63 de la plaie 3 en fonction du nombre de parties distinctes de la plaie 3.

Le module de traitement 61 est également agencé pour affecter à la zone ou chaque zone délimitée par un contour 63 une phase d'évolution a, b, c. Cette détermination peut être réalisée par une méthode de segmentation de type ligne de partage des eaux ou de croissance de région.

Alternativement une méthode de motifs binaires locaux ou local binary patterns en anglais peut être utilisée par le module de traitement 61.

Lorsque la ou les zones sont déterminées, le module de traitement 61 affecte à la ou les zones une phase d'évolution a, b, c propre. Une phase d'évolution a, b, c est définie par une pluralité d'éléments descriptifs comme une couleur et/ou une texture.

La phase d'évolution a, b, c est déterminée par une méthode de régression des moindres carrés partiels de Kernel ou KPLS, le résultat étant une probabilité d'appartenance à une phase d'évolution a, b, c.

Alternativement une méthode de classification de type Software Method Machine ou Deep Learning peut être utilisée par le module de traitement.

Le module de traitement 61 est également agencé pour mettre en œuvre une phase initiale, c'est-à-dire préalable au fonctionnement, pendant laquelle une pluralité d'informations relatives à des phases d'évolution sont transmises au module de détermination avec pour chaque information une correspondance avec une phase d'évolution a, b, c donnée.

En outre et comme illustré à la figure 7, le système de génération 5 d'un mélange gazeux comprend un système de mesure 67 agencé pour mesurer une oxymétrie transcutanée de la plaie 3 et agencé pour transmettre des valeurs de mesures au processeur 41.

Le but est de renseigner le processeur 41 sur l'état de la plaie 3 en déterminant un état réel de l'état d'oxygénation des tissus de la plaie 3. L'oxymétrie transcutanée est également connue sous la dénomination TCPO2.

Selon les variantes de réalisation, le système de mesure 67 peut en outre comprendre un dispositif de capillaroscopie et/ou un capteur de température de la plaie.

Ainsi le module de traitement 61 est agencé pour centraliser les informations de mesure dans le but de les exploiter. Le module de traitement 61 est également agencé pour déterminer une consigne de traitement à partir de toutes ces informations.

La consigne de commande définit la fonction de variation temporelle de la pression FVTP et/ou la fonction de variation temporelle de la fraction molaire de dioxygène FVTO2.

Le module de traitement 61 permet ainsi suite à l'analyse des valeurs mesurées de déterminer par calcul la consigne de commande la plus adaptée à l'état de la plaie 3.

La consigne de commande définit pour le mélange gazeux un degré d'hygrométrie, une température T, une fraction molaire de vapeur d'eau H2O, une fraction molaire de diazote N2, une fraction molaire de dioxyde de carbone CO2, et/ou une fraction de gaz additionnel ou aérosol 51.

L'interface utilisateur 53 est configurée pour soumettre à une validation de l'utilisateur la consigne de commande déterminée par le module de traitement 61.

Ainsi la consigne de commande est à considérer comme une proposition faite au médecin responsable du traitement qui peut ensuite éventuellement modifier la consigne de commande si son analyse de l'état de la plaie 3 est différente.

Il apparait donc que le module de traitement 61 considère l'état réel de plaie ainsi que son évolution pour déterminer quel est le traitement le plus adapté. Le fait de configurer l'interface 53 pour proposer un traitement permet au médecin de gagner du temps s'il se rend compte que son analyse est identique.

Dans ce cas, le médecin n'a plus qu'à valider la proposition du module de traitement 61.

## Revendications

1. Système de génération (5) d'un mélange gazeux pour une enceinte de traitement (7) d'une plaie (3) comprenant :
- un générateur de mélange gazeux (11) apte à délivrer dans l'enceinte de traitement (7) ledit mélange gazeux à un débit déterminé, en faisant varier une fraction molaire de dioxygène (O2) dudit mélange gazeux délivré et en faisant varier une fraction molaire de vapeur d'eau (H2O) dudit mélange gazeux délivré,
- une unité de contrôle (39) du mélange gazeux délivré pourvue d'un processeur (41) apte à recevoir une information relative à une pression (P) interne de l'enceinte de traitement (7) et/ou agencé pour déterminer un débit dudit mélange gazeux à délivrer dans l'enceinte de traitement (7) pour atteindre une pression (P) interne cible,
la pression (P) cible étant définie par une fonction de variation temporelle de la pression (FVTP) entre une pression minimale correspondant à une pression ambiante externe à l'enceinte de traitement (7) et une pression maximale,
le processeur (41) étant en outre agencé pour déterminer une fonction de variation temporelle de la fraction molaire de dioxygène (FVTO2) du mélange gazeux à délivrer et une fonction de variation temporelle de la fraction molaire de vapeur d'eau (FVTH2O) du mélange gazeux à délivrer,
**caractérisé en ce que** l'unité de contrôle (39) comprend un capteur apte à déterminer une information relative à une dimension (a1, a2, a3) et/ou à une phase d'évolution (a, b, c) d'au moins une partie d'une plaie (3), le capteur comprenant une caméra,
**en ce que** le processeur (41) est agencé pour recevoir ladite information relative à une dimension (a1, a2, a3) et/ou à une phase d'évolution (a, b, c) d'au moins une partie d'une plaie (3),
et **en ce que** le processeur (41) comprend un module de traitement qui détermine une phase d'évolution de tout ou partie de la plaie, la détermination étant réalisée à partir de l'information relative à la dimension (a1, a2, a3) et/ou à la phase d'évolution (a, b, c) d'au moins une partie de la plaie (3),
la phase d'évolution (a, b, c) étant définie par une pluralité d'éléments descriptifs.

2. Système de génération (5) d'un mélange gazeux selon la revendication 1, dans lequel le générateur de mélange gazeux (11) délivre dans l'enceinte de traitement ledit mélange gazeux à un degré d'hygrométrie (DHH2O) déterminé.

3. Système de génération (5) d'un mélange gazeux selon l'une des revendications 1 ou 2, dans lequel le générateur de mélange gazeux (11) délivre dans l'enceinte de traitement (7) ledit mélange gazeux à une température (T) déterminée.

4. Système de génération (5) d'un mélange gazeux selon l'une des revendications 1 à 3, dans lequel le mélange gazeux comprend en outre une fraction molaire de diazote (N2) et/ou une fraction molaire de dioxyde de carbone (CO2) et/ou un autre gaz adapté.

5. Système de génération (5) d'un mélange gazeux selon l'une des revendications 1 à 4, dans lequel l'unité de contrôle (39) comprend une interface utilisateur (53) pourvue d'une commande utilisateur (55) et/ou d'un organe de retour (57) comprenant un écran et/ou un voyant.

6. Système de génération (5) d'un mélange gazeux selon l'une des revendications 1 à 5 comprenant un système de mesure (67) d'une oxymétrie transcutanée de la plaie agencé pour transmettre des valeurs de mesures au processeur (41).

7. Système de génération (5) d'un mélange gazeux selon la revendication 6, dans lequel le système de mesure (67) comprend en outre un dispositif de capillaroscopie et/ou un capteur de température de la plaie (3).

8. Système de génération (5) d'un mélange gazeux selon l'une des revendications 1 à 7, dans lequel le module de traitement (61) détermine un contour (63) de tout ou partie de la plaie (3) et affecte à une zone délimitée par ledit contour (63) une phase d'évolution (a, b, c), l'affectation étant réalisée à partir de l'information relative à une dimension (a1, a2, a3) et/ou à une phase d'évolution (a, b, c) d'au moins une partie d'une plaie (3).

9. Système de génération (5) d'un mélange gazeux selon l'une des revendications 1 à 7, dans lequel le module de traitement (61) compare l'information relative à une dimension (a1, a2, a3) et/ou à une phase d'évolution (a, b, c) d'au moins une partie d'une plaie (3) à une pluralité d'informations de définitions de phases d'évolution pour déterminer la phase d'évolution (a, b, c) de tout ou partie de la plaie (3).

10. Système de génération (5) d'un mélange gazeux selon l'une des revendications 1 à 9, dans lequel le module de traitement (61) détermine une consigne de commande définissant la fonction de variation temporelle de la pression (FVTP) et/ou la fonction de variation temporelle de la fraction molaire de dioxygène (FVTO2).

11. Appareil de traitement (1) comprenant un système de génération (5) selon l'une des revendications 1 à 10 et une enceinte de traitement (7) disposée sur et/ou autour d'une partie d'un corps d'un utilisateur de manière à engendrer une zone de traitement (29).

12. Système de génération (5) d'un mélange gazeux selon l'une des revendications 1 à 3, dans lequel la fonction de variation temporelle de la pression (FVTP) ou la fonction de variation temporelle de la fraction molaire de dioxygène ou la fonction de variation temporelle de la fraction molaire de vapeur d'eau est une fonction périodique.

13. Système de génération (5) d'un mélange gazeux selon la revendication précédente, dans lequel la fonction de variation temporelle de la pression (FVTP) ou la fonction de variation temporelle de la fraction molaire de dioxygène ou la fonction de variation temporelle de la fraction molaire de vapeur d'eau est une fonction périodique de type sinusoïdal, triangulaire ou présentant des échelons.

## Patentansprüche

1. System zum Erzeugen (5) eines Gasgemisches für eine Behandlungskammer (7) einer Wunde (3), umfassend:
- einen Gasgemischgenerator (11), der geeignet ist, das Gasgemisch mit einer bestimmten Durchflussmenge in die Behandlungskammer (7) zuzuführen, indem eine molare Fraktion von Disauerstoff (02) des zugeführten Gasgemisches variiert wird und indem eine molare Fraktion von Wasserdampf (H2O) des zugeführten Gasgemisches variiert wird,
- eine Steuereinheit (39) des zugeführten Gasgemisches, die mit einem Prozessor (41) versehen ist, der geeignet ist, eine Information über einen Innendruck (P) der Behandlungskammer (7) zu empfangen und/oder ausgelegt ist, um eine Durchflussmenge des Gasgemisches zu bestimmen, die in die Behandlungskammer (7) zuzuführen ist, um einen Sollinnendruck (P) zu erreichen,
wobei der Solldruck (P) durch eine Funktion der zeitlichen Variation des Drucks (FVTP) zwischen einem Mindestdruck, der einem Umgebungsdruck außerhalb der Behandlungskammer (7) entspricht, und einem Maximaldruck definiert wird,
wobei der Prozessor (41) ferner ausgelegt ist, um eine Funktion der zeitlichen Variation der molaren Fraktion von Disauerstoff (FVTO2) des Gasgemisches, das zuzuführen ist, und eine Funktion der zeitlichen Variation der molaren Fraktion von Wasserdampf (FVTH2O) des Gasgemisches, das zuzuführen ist, zu bestimmen,
**dadurch gekennzeichnet, dass** die Steuereinheit (39) einen Sensor aufweist, der geeignet ist, eine Information über eine Abmessung (a1, a2, a3) und/oder eine Entwicklungsphase (a, b, c) von mindestens einem Teil einer Wunde (3) zu bestimmen, wobei der Sensor eine Kamera aufweist,
dadurch, dass der Prozessor (41) ausgelegt ist, um die Information über eine Abmessung (a1, a2, a3) und/oder eine Entwicklungsphase (a, b, c) von mindestens einem Teil einer Wunde (3) zu empfangen,
und dadurch, dass der Prozessor (41) ein Verarbeitungsmodul aufweist, das eine Entwicklungsphase der ganzen oder eines Teils der Wunde bestimmt, wobei das Bestimmen ausgehend von der Information über die Abmessung (a1, a2, a3) und/oder die Entwicklungsphase (a, b, c) von mindestens einem Teil der Wunde (3) durchgeführt wird,
wobei die Entwicklungsphase (a, b, c) durch mehrere beschreibende Elemente definiert wird.

2. System zum Erzeugen (5) eines Gasgemisches nach Anspruch 1, wobei der Gasgemischgenerator (11) das Gasgemisch mit einer bestimmten Luftfeuchtigkeit (DHH2O) in die Behandlungskammer (7) zuführt.

3. System zum Erzeugen (5) eines Gasgemisches nach einem der Ansprüche 1 oder 2, wobei der Gasgemischgenerator (11) das Gasgemisch bei einer bestimmten Temperatur (T) in die Behandlungskammer (7) zuführt.

4. System zum Erzeugen (5) eines Gasgemisches nach einem der Ansprüche 1 bis 3, wobei das Gasgemisch ferner eine molare Fraktion von Distickstoffoxid (N2) und/oder eine molare Fraktion von Kohlendioxid (CO2) und/oder ein anderes geeignetes Gas umfasst.

5. System zum Erzeugen (5) eines Gasgemisches nach einem der Ansprüche 1 bis 4, wobei die Steuereinheit (39) eine Benutzerschnittstelle (53) aufweist, die mit einer Benutzersteuerung (55) und/oder einem Rückschlagorgan (57) versehen ist, umfassend einen Bildschirm und/oder eine Kontrollleuchte.

6. System zum Erzeugen (5) eines Gasgemisches nach einem der Ansprüche 1 bis 5, umfassend ein Messsystem (67) einer transkutanen Oximetrie der Wunde, das ausgebildet ist, um Messwerte an den Prozessor (41) zu senden.

7. System zum Erzeugen (5) eines Gasgemisches nach Anspruch 6, wobei das Messsystem (67) ferner ein Kapillaroskopiegerät und/oder einen Wundtemperatursensor (3) aufweist.

8. System zum Erzeugen (5) eines Gasgemisches nach einem der Ansprüche 1 bis 7, wobei das Verarbeitungsmodul (61) eine Kontur (63) der gesamten oder eines Teils der Wunde (3) bestimmt und einem Bereich, der durch diese Kontur (63) begrenzt ist, eine Entwicklungsphase (a, b, c) zuordnet, wobei das Zuordnen ausgehend von der Information über eine Abmessung (a1, a2, a3) und/oder eine Entwicklungsphase (a, b, c) von mindestens einem Teil einer Wunde (3) durchgeführt wird.

9. System zum Erzeugen (5) eines Gasgemisches nach einem der Ansprüche 1 bis 7, wobei das Verarbeitungsmodul (61) die Information über eine Abmessung (a1, a2, a3) und/oder eine Entwicklungsphase (a, b, c) von mindestens einem Teil einer Wunde (3) mit mehreren Informationen von Definitionen von Entwicklungsphasen vergleicht, um die Entwicklungsphase (a, b, c) der gesamten oder eines Teils der Wunde (3) zu bestimmen.

10. System zum Erzeugen (5) eines Gasgemisches nach einem der Ansprüche 1 bis 9, wobei das Verarbeitungsmodul (61) einen Steuersollwert bestimmt, der die Funktion der zeitlichen Variation des Drucks (FVTP) und/oder die Funktion der zeitlichen Variation der molaren Fraktion von Disauerstoff (FVTO2) definiert.

11. Behandlungsvorrichtung (1), umfassend ein System zum Erzeugen (5) nach einem der Ansprüche 1 bis 10 und eine Behandlungskammer (7), die an und/oder um einen Teil eines Körpers eines Benutzers derart angeordnet ist, um eine Behandlungszone (29) zu erzeugen.

12. System zum Erzeugen (5) eines Gasgemisches nach einem der Ansprüche 1 bis 3, wobei die Funktion der zeitlichen Variation des Drucks (FVTP) oder die Funktion der zeitlichen Variation der molaren Fraktion von Disauerstoff oder die Funktion der zeitlichen Variation der molaren Fraktion von Wasserdampf eine periodische Funktion ist.

13. System zum Erzeugen (5) eines Gasgemisches nach dem vorhergehenden Anspruch, wobei die Funktion der zeitlichen Variation des Drucks (FVTP) oder die Funktion der zeitlichen Variation der molaren Fraktion von Disauerstoff oder die Funktion der zeitlichen Variation der molaren Fraktion von Wasserdampf eine periodische Funktion des Typs der sinusförmigen, dreieckigen oder stufenförmigen Funktion ist.

## Claims

1. A system (5) for generating a gaseous mixture for a treatment enclosure (7) of a wound (3) comprising:
- a gaseous mixture generator (11) able to deliver said gaseous mixture to the treatment enclosure (7) at a defined flow rate, by varying a molar fraction of dioxygen (02) of said delivered gaseous mixture and by varying a molar fraction of water vapor (H2O) of said delivered gaseous mixture,
- a control unit (39) for controlling the delivered gaseous mixture provided with a processor (41) able to receive data relating to a pressure (P) inside the treatment enclosure (7) and/or designed to determine a flow rate of said gaseous mixture to be delivered inside the treatment enclosure (7) in order to obtain an internal target pressure (P),
the target pressure (P) being defined by a time variation function of the pressure (TVFP) between a minimum pressure, corresponding to an ambient pressure outside the treatment enclosure (7), and a maximum pressure,
the processor (41) also being designed to determine a time variation function of the molar fraction of dioxygen (TVFO2) of the gaseous mixture to be delivered and a time variation function of the molar fraction of water vapor (TVFH2O) of the gaseous mixture to be delivered
**characterized in that** the control unit (39) comprises a sensor able to determine a data relating to a dimension (a1, a2, a3) and/or the development phase (a, b, c) of at least a part of the wound (3), said sensor comprising a video camera,
**in that** the processor (41) is configured to receive said data relating to a dimension (a1, a2, a3) and/or to a development phase (a, b, c) of at least a part of a wound (3),
and **in that** the processor (41) comprises a treatment module that determines a development phase of all or part of the wound, the determination being carried out based on the data relating to the dimension (a1, a2, a3) and/or the development phase (a, b, c) of at least a part of the wound (3),
the development phase (a, b, c) being defined by a plurality of descriptive elements.

2. System (5) for generating a gaseous mixture according to claim 1, wherein the gaseous mixture generator (11) delivers said gaseous mixture to the treatment enclosure at a defined hygrometry degree (HDH2O).

3. System (5) for generating a gaseous mixture according to one of claims 1 or 2, wherein the gaseous mixture generator (11) delivers said gaseous mixture to the treatment enclosure (7) at a defined temperature (T).

4. System (5) for generating a gaseous mixture according to one of claims 1 to 3, wherein the gaseous mixture also comprises a molar fraction of dinitrogen (N2) and/or a molar fraction of carbon dioxide (CO2) and/or another suitable gas.

5. System (5) for generating a gaseous mixture according to one of claims 1 to 4, wherein the control unit (39) comprises a user interface (53) provided with a user control (55) and/or a feedback device (57) comprising a screen and/or an indicator light.

6. System (5) for generating a gaseous mixture according to one of claims 1 to 5 comprising a measurement system (67) of a transcutaneous oximetry of the wound configured to transmit the measurement values to the processor (41).

7. System (5) for generating a gaseous mixture according to claim 6, wherein the measurement system (67) also comprises a capillaroscopy device and/or a temperature sensor of the wound (3).

8. System (5) for generating a gaseous mixture according to one of claims 1 to 7, wherein the treatment module (61) determines a contour (63) of all or part of the wound (3) and
assigns, to an area delimited by said contour (63), a development phase (a, b, c), the assignment being carried out based on the data relating to a dimension (a1, a2, a3) and/or a development phase (a, b, c) of at least a part of a wound (3).

9. System (5) for generating a gaseous mixture according to one of claims 1 to 7, wherein the treatment module (61) compares the data relating to a dimension (a1, a2, a3) and/or a development phase (a, b, c) of at least a part of a wound (3) with a plurality of data on definitions of development phases for determining the development phase (a, b, c) of all or part of the wound (3).

10. System (5) for generating a gaseous mixture according to one of claims 1 to 9, wherein the treatment module (61) determines a control set point defining the time variation function of the pressure (TVFP) and/or the time variation function of the molar fraction of dioxygen (TVFO2)

11. A treatment device (1) comprising a generating system (5) according to one of claims 1 to 10 and a treatment enclosure (7) disposed on and/or around a body part of a user so as to produce a treatment area (29).

12. System (5) for generating a gaseous mixture according to one of claims 1 to 3, wherein-the time variation function of the pressure (TVFP) or the time variation function of the molar fraction of dioxygen or the time variation function of the molar fraction of water vapor is a periodic function.

13. System (5) for generating a gaseous mixture according to the preceding claim, wherein the time variation function of the pressure (TVFP) or the time variation function of the molar fraction of dioxygen or the time variation function of the molar fraction of water vapor is a periodic function of sinusoidal, triangular, or step type.
